# EUROPEAN PATENT APPLICATION

(11) **EP 1 354 644 A2**
(43) Date of publication of application: **22.10.2003**
(21) Application number: 02380175.6
(22) Date of filing: 01.08.2002
(51) Int. Cl.: B09B 3/00, C05F 11/02, C12P 5/02

(54) **Tank for the decomposing of animal bodies**

(30) Priority: 15.04.2002 ES 200200931 U
(71) Applicant: Molina Benitez, Josep Antoni, 08271 Artés (Barcelona) (ES)
(72) Inventor: Molina Benitez, Josep Antoni, 08271 Artés (Barcelona) (ES)
(74) Representative: Matamoron Hernandez, José Pedro

(57) **Abstract**

This tank is made up of a closed container (1,1') which comprises of: an opening (2) with a lid (3), an enzyme-bacteria doser (4), an opening with lid (5) for the introduction of a suction pipe (6) to remove the waste liquids from the bottom of the container (1 or 1'), some heating elements (7) aimed at maintaining the internal temperature of the container (1 or 1'), and some complementary devices optionally made up of an air inlet (8) for the addition of air to the inside of the container (1) by means of forced air (9), or by a wind driven exhaust (10) for the removal of the gases produced.

## Description

### AIM OF THE INVENTION

The invention refers to a tank for the decomposing of the bodies of animals. It is applicable to livestock installations where animals are bred and/or fattened. The purpose of this tank is to make it easier for the disposal of the remains being of animal origin and dead animals from varying causes.

### BACKGROUND TO THE INVENTION

At the present time, one of the problems posed at livestock installations is the disposal of organic remains or bodies of animals, ensuring appropriate hygienic conditions and compliance with the legislation in force.

Previously, the livestock installations disposed of the animal remains and bodies by simply burying them. This practice is now prohibited due to the possibility of the contamination of underground waters. It must be taken into account that the pollution of the waters apart from not being desirable could be very dangerous in the event of the bodies coming from sick animals.

Another of the methods used by the livestock companies at the present time for the disposal of animal bodies is by using the method of collection by authorised companies. These companies remove the animals from the farms for disposal by means of suitable processes. This system of disposal, although seeming to be convenient for the farmer, in practice is not. This is because it represents a dependency on an outside service that does not always fulfil the collection times, and in this case, in dealing with merchandise of this type, a delay might represent big problems in respect of hygiene and/or health.

Another system used at the present time for the disposal of bodies is the construction of tanks from bricks or concrete in which the animal remains are introduced, but due to the materials used in the construction, they are not watertight, and therefore leaks can occur. The problem of these types of tanks becomes worse if it is taken into account that on many occasions it is essential to pour in aggressive products in order to reduce the decomposition time of the bodies, with which, the leaks that could be caused are absolutely undesirable.

### DESCRIPTION OF THE INVENTION

In order to solve the above-mentioned problems the tank object of the invention has been thought up for the decomposing of animal bodies that has certain constructive peculiarities aimed at making the operation easier, ensuring correct water-tightness, avoiding the possibility of leaks and complying with all the legal and hygiene regulations in force.

The tank for the decomposing of the bodies of animals is applicable to livestock installations where animals are bred and/or fattened. The purpose of this tank is for the disposal of the remains being of animal origin and dead animals from varying causes.

In accordance with the invention, the tank is comprised of a closed container made from an anti-corrosive material that is thermo-stable and leak proof, such as glass fibre with polyester, or any other type of material being plastic or otherwise. The fact of it being thermo-stable guarantees that its physical shape will not be altered with the addition of heat.

The stated container can have differing sizes in line with the size of the livestock installation where it is to be used. It will include an opening with a lid for the introduction of the bodies or remains to be disposed of, a doser for the enzymes-bacteria to carry out the introduction of same to the inside, an opening with a lid in order to allow the introduction of a suction pipe to be able to suck out the waste liquids from the bottom of the container when necessary, some heating elements to keep the inside temperature constant or suitable for the decomposition process needs of the bodies and some complementary devices aimed both at improving the process for the break down of the bodies and remains and equally to reduce the time used for said process. This break down can be optionally in an aerobic or anaerobic manner.

The doser for the enzymes-bacteria allows the use of chemical formulations that are presented both in a solid or equally liquid form. In turn, the dosing can optionally be carried out manually, semiautomatically or automatically, at all times the consumption being controlled.

The complementary devices aimed at improving the decomposition process of the bodies in an aerobic manner will have an air inlet to allow the addition of air to the inside of the container by means of forced ventilation.

These forced air ventilation devices can be fans, blowers or any other mechanical means, diffusers, nozzles etc., can also be installed. Finally, any element that will help to improve the transfer of oxygen will be valid.

On the other hand, the complementary devices aimed at improving the process for the break down of the bodies in an anaerobic manner will be a wind driven exhaust mechanism to carry out the evacuation of the gases produce, as by helping the ventilation improves the performance of the equipment. Obviously, a wind driven exhaust mechanism is used by its autonomy but any other exhaust mechanism will also be valid.

The heating elements aimed at maintaining the inside of the container at a constant temperature or that which is suitable to the process can be an electrical element, a coil of hot water, steam, thermal oil or any other similar device. In addition, said elements allow the temperature to be controlled by a thermostat that has an indicator on the outside of the container.

The tank described for the breaking down of bodies has advantages over the systems at present being used: their water-tightness, mechanical resistance and resistance to corrosion, their ease of installation due mainly to the reduced weight of the assembly and finally and importantly giving independence to the livestock operation from the companies dedicated to the disposal of organic waste.

The water-tightness is provided both because of the waterproof nature of the materials used for the construction of the container and also because of being manufactured from one single piece, which avoids leaks to the ground where it is installed.

The mechanical resistance of the container is significant in so far as it avoids a possible collapsing due to the load placed on it by the land in which it is installed.

The container offers resistance due to corrosion due to the nature of the materials used in its manufacture. This will ensure chemical resistance of the assembly, even against the final pouring in of any highly corrosive chemical product.

In addition, the complementary devices fitted to the tank make the accelerated break down of the bodies and animal remains easier.

The versatility of these tanks allows easy adaptation to the needs of each place of operation. The number of units necessary at each livestock location being in line not only with the livestock farm being dealt with, but of the livestock capacity authorised at each one of them, and of the capacity given by each one of these tanks that are installed. All the factors are subordinate to that which is established in the regulations in force for each region.

### DESCRIPTION OF THE DRAWINGS

In order to complement the description that is being made and for the purpose of making the understanding of the invention features easier, the present descriptive memorandum is accompanied by a set of drawings in which, by way of illustration and without limitation, the following has been represented:

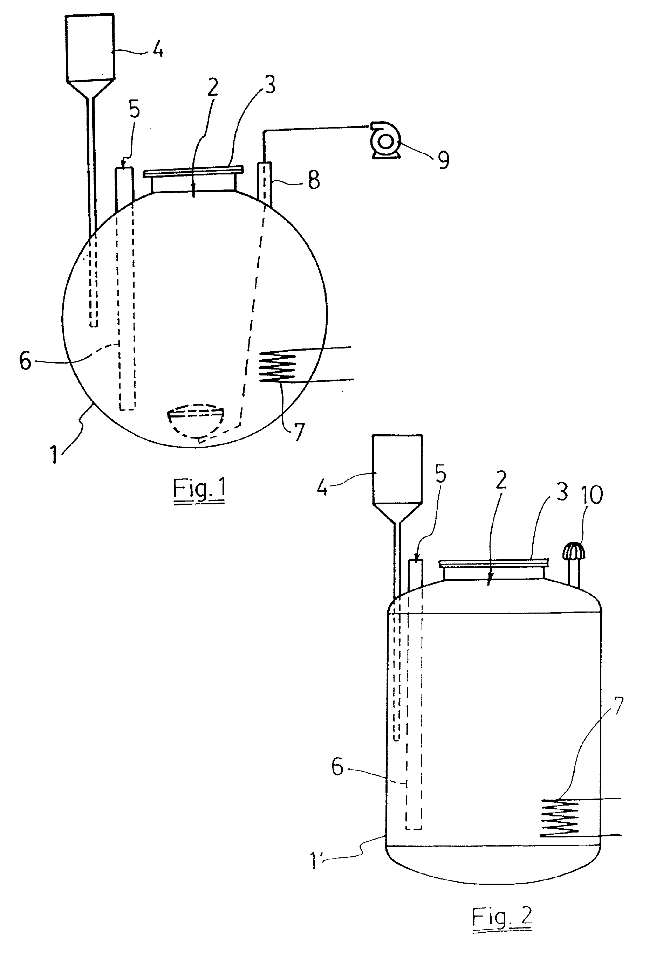

Figure 1 shows a diagrammatic view in elevation of a tank for the breaking down of animal bodies in an aerobic manner.
Figure 2 shows a diagrammatic view in elevation of a tank for the breaking down of animal bodies in an anaerobic manner.

### PREFERRED EMBODIMENT OF THE INVENTION

As can be seen in the referenced drawings, there are two different ways of carrying out the tanks in accordance with the decomposition process for animal bodies and animal remains depending on the preference for the aerobic or anaerobic method.

In both methods, the tank is made up of a closed container (1 or 1') manufactured from fibre glass and polyester reinforced with resins that have high chemical resistance to acids and alkalis and with resins which provide the layers with high levels of mechanical resistance.

The container (1, 1') in both cases includes an inlet (2) for the introduction of the bodies or animal remains to the inside and a lid (3) to close the container.

An enzyme-bacteria doser (4) allows the addition of these chemical products in appropriate amounts to the inside of the container (1,1').

The tank (1,1') has an opening with a lid (5) to allow the introduction of a suction pipe (6), which will allow the emptying of the waste liquids from the bottom of the tank (1,1') when necessary.

The tank (1,1') also has some means of heating, shown in the example of the carrying out by an electrical element (7) which will allow the inside of the tank (1 or 1') to be kept at a controlled temperature by means of a thermostat.

The closed container (1) for the breaking down of the bodies in an aerobic manner, shown in drawing 1, has a spherical shape, and has an air inlet (8) to allow the addition of air to the inside of the container (1) by means of forced ventilation elements (9).

The closed container (1') for the breaking down of the bodies in an anaerobic manner, shown in drawing 2, is cylindrical in shape with a domed base, and has a wind driven exhaust mechanism (10) to carry out the removal of the gases produced and to improve the decomposition process of the bodies.

Once having described the nature of the invention sufficiently, likewise an example of the preferred method of carrying it out, it is placed on record for such suitable purposes that the materials, shape, size and layout of the elements described can be modified, provided that it does not mean a change to the essential characteristics of the invention that is claimed below.

## Claims

1. Tank for the decomposition of animal bodies, being of the type used in livestock installations where animals are bred and/or fattened; the purpose of this tank is to make it easier for the disposal of the remains being of animal origin and dead animals from varying causes; **characterised in that** it is made up of a closed container (1,1') made from an anti-corrosive, thermo-stable and waterproof material, which comprises of: an inlet (2) with a lid (3) for the introduction of the animal bodies and remains, an enzyme-bacteria doser (4) to carry out the introduction of them into the container (1 or 1'), an opening with a lid (5) for the introduction of a suction pipe (6) for the suction of the waste liquids from the bottom of the tank (1 or 1'), some heating elements (7) aimed at keeping the inside of the tank (1 or 1') at a constant temperature or a temperature that is suitable for the needs of the animal body decomposition process, and some complementary devices aimed at improving the break down of the animal bodies in an aerobic or anaerobic manner.

2. Tank, in accordance with the above claim, is **characterised in that** the heating elements (7) aimed at keeping the inside of the tank (1 or 1') at a constant temperature or a temperature that is suitable for the needs of the animal body decomposition process allows the control by means of a thermostat that has an indicator and/or warning on the outside.

3. Tank, in accordance with the above claims, is **characterised in that** the complementary devices fitted to the tank (1) to improve the breakdown of the animal bodies in an aerobic manner is comprised of an air inlet (8) for the addition of air to the inside of the container (1) by means of forced air devices (9).

4. Tank, in accordance with the above claims 1 and 2, is **characterised in that** the complementary devices fitted to the tank (1') to improve the breakdown of the animal bodies in an anaerobic manner is comprised of a wind driven exhaust (10) for the removal of gases produced.
